(19) Europäisches Patentamt
European Patent Office
Office européen des brevets

(11) **EP 0 842 654 A2**

(12) **EUROPÄISCHE PATENTANMELDUNG**

(43) Veröffentlichungstag:
**20.05.1998 Patentblatt 1998/21**

(51) Int. Cl.⁶: **A61K 7/06**

(21) Anmeldenummer: **97118477.5**

(22) Anmeldetag: **24.10.1997**

(84) Benannte Vertragsstaaten:
**AT BE CH DE DK ES FI FR GB GR IE IT LI LU MC NL PT SE**
Benannte Erstreckungsstaaten:
**AL LT LV RO SI**

(30) Priorität: **19.11.1996 DE 19647765**

(71) Anmelder:
**Wella Aktiengesellschaft**
**64274 Darmstadt (DE)**

(72) Erfinder:
- **Eicken, Ulrich**
  **1700 Fribourg (CH)**
- **Kunz, Manuela, Dr.**
  **1723 Marly (CH)**
- **Jungo, Sybille**
  **1723 Marly (CH)**

(54) **Haarpflegemittel mit langdauernder Wirkung**

(57)

a) 0,1 bis 20 Gewichts-% eines Carriers, der mindestens eine Hydroxylgruppe im Molekül aufweist, bei einem pH-Wert von 7,0 ungeladen ist, einen Octanol-Wasser-Verteilungskoeffizienten (log P) von 0,3 bis 3,0 besitzt, jedoch nicht 2-Benzyloxyethanol ist und

b) 0,1 bis 20 Gewichts-% eines oder mehrerer kosmetisch akzeptabler, kationischer oder amphoterer Polymerer.

EP 0 842 654 A2

## Beschreibung

Gegenstand der Erfindung ist ein Haarpflegemittel mit langdauernder Wirkung, bei dem die konditionierende Wirkung polymerer Verbindungen durch den Zusatz eines speziellen Carriers verstärkt und verlängert wird.

Es ist bekannt, daß durch den Zusatz bestimmter polymerer Verbindungen die konditionierenden Wirkungen von Haarpflegemitteln erheblich verbessert werden können. Sie können beispielsweise die Kämmbarkeit, die Formbarkeit und den Glanz von Haaren beträchtlich erhöhen, jedoch lassen diese Wirkungen schon bald wieder nach. Es hat deshalb schon verschiedentlich Bemühungen gegeben, die durch polymere Zusatzstoffe erzielbaren Effekte von Haarpflegemitteln zu verstärken und zu verlängern.

So ist bereits aus dem europäischen Patent 470 381 bekannt, daß die konditionierende Wirkung eines Haarpflegemittels, welches kationische oder amphotere Polymere enthält, erheblich verlängert und verstärkt wird, wenn es den als Penetrationsbeschleuniger oder auch Carrier bezeichneten 2-Benzyloxyethanol enthält. Allerdings können derartige Carrier gelegentlich zu Reizungen der Kopfhaut oder zu allergischen Reaktionen führen. Außerdem hängt ihre Wirksamkeit als Penetrationsbeschleuniger auch von den Eigenschaften der übrigen im Haarpflegemittel enthaltenen Komponenten ab, die sehr stark variieren können. Deshalb ist es wünschenswert, eine große Anzahl unterschiedlicher Carrier zur Verfügung zu haben, um den jeweiligen toxikologischen und kosmetischen Erfordernissen entsprechende optimale Rezepturen von Haar-pflegemitteln zur Verfügung stellen zu können.

Diese Aufgabe wird erfindungsgemäß durch ein Haarpflegemittel mit langdauernder Wirkung gelöst, das in einer kosmetisch anwendbaren Basiszubereitung

a) 0,1 bis 20 Gewichts-% eines Carriers, der mindestens eine Hydroxylgruppe im Molekül aufweist, bei einem pH-Wert von 7,0 ungeladen ist, einen Octanol-Wasser-Verteilungskoeffizienten (log P) von 0,3 bis 3,0 besitzt, jedoch nicht 2-Benzyloxyethanol ist und

b) 0,1 bis 20 Gewichts-% eines oder mehrerer kosmetisch akzeptabler, anionischer, kationischer oder amphoterer Polymerer enthält.

Besonders bevorzugt sind Haarpflegemittel, bei denen der Carrier einen Octanol-WasserVerteilungskoeffizienten (log P) von 0,5 bis 2,7 aufweist.

Der Octanol-Wasser-Verteilungskoeffizient (log P) ist ein Maß für die Verteilung einer Substanz zwischen der wäßrigen und der organischen Phase (hier: Octanol) und wird wie folgt definiert:

$$\log P = \log\left(\frac{[\text{Substanz}]\text{Octanol}}{[\text{Substanz}]\text{Wasser}}\right)$$

Beispiele für berechnete und gerechnete log P-Werte findet man in A. Leo, C. Hansch, D. Elkins, Chemical Reviews, Volume 71, Nr. 6, (1971). Je höher der log P-Wert liegt, desto hydrophober ist die Substanz. Eine Verbindung mit einem log P-Wert unter 0 ist hydrophil und besser in der wäßrigen als in der organischen Phase löslich. Eine Verbindung mit einem log P-Wert von 1 ist zehnmal so gut in der organischen Phase löslich wie in der wäßrigen und eine Verbindung mit einem log P-Wert von 2 ist hundertmal so gut in der organischen Phase löslich wie in der wäßrigen.

Geeignete Carrier sind Verbindungen der Formel (I)

$$(R)_p - Z - X - Y - OH \qquad (I),$$

wobei X eine $(CH_2)_n$-Gruppe mit n = 0, 1 oder 2, eine Alkoxygruppe, eine Hydroxyalkylgruppe, Sauerstoff, oder Schwefel bedeutet, Y eine $(CH_2)_m$-Gruppe mit m = 0, 1 oder 2 bedeutet; Z einen 5- bis 8-gliedrigen aliphatischen oder aromatischen Carbozykıus oder Heterozyklus bedeutet und $(R)_p$ für bis zu 5 Substituenten (p = 0, 1, 2, 3, 4 oder 5) steht, welche unabhängig voneinander Wasserstoff, eine Hydroxygruppe, eine Alkylgruppe, eine Alkoxygruppe, eine Acetamido-gruppe, eine Formylgruppe oder eine Formylalkylgruppe darstellen, unter der Voraussetzung, daß die Verbindung der Formel (I) nicht gleich 2-Benzyloxyethanol ist.

Geeignet sind auch $\alpha$-Hydroxycarbonsäureester der Formel (II)

$$R^1 - CH(OH) - COOR^2 \qquad (II),$$

in der $R^1$ und $R^2$ für gleiche oder verschiedene Alkylgruppen mit 1 bis 6 Kohlenstoffatomen stehen, wobei jede Alkylgruppe durch 1 oder 2 Sauerstoffatome mit Etherfunktion unterbrochen sein kann.

Bevorzugte Carrier der Formeln (I) und (II) sind Vanillin (4-Hydroxy-3-methoxy-benzaldehyd), p-Hydroxyanisol, 3-Hydroxy-4-methoxy-benzaldehyd, 2-Phenoxyethanol, Salicylaldehyd, 3,5-Dihydroxybenzaldehyd, 3,4-Dihydroxybenzaldehyd, 4-Hydroxphenylacetamid, p-Hydroxybenzoesäure-methylester, p-Hydroxybenzaldehyd, m-Kresol, Hydrochinon-monomethylether, o-Fluorphenol, m-Fluorphenol, p-Fluorphenol, 2-(2'-Hydroxyphenoxy)-ethanol, 3,4-Methylendioxy-phenol, Resorcin-monomethylether, 3,4-Dimethoxy-phenol, 3-Trifluormethyl-phenol, Resorcin-monoacetat, Ethylvanillin, 2-Thiophenethanol, Milchsäurebutylester und Glykolsäurebutylester, wobei Vanillin, allein oder in Kombination mit anderen Carriern, besonders bevorzugt ist. Der Carrier wird vorzugsweise in einer Menge von 0,1 bis 20 Gewichts-%, insbesondere 1 bis 9 Gewichts-%, eingesetzt.

In dem erfindungsgemäßen Haarpflegemittel kommen kosmetisch akzeptable, anionische, kationische oder amphotere Polymere zur Anwendung.

Das in dem erfindungsgemäßen Haarpflegemittel enthaltene anionische Polymere kann entweder ein natürliches oder ein synthetisches Polymeres sein.

Beispiele für natürliche anionische Polymere sind Xanthan-Gummi, Carrageen, Alginate, Pektin, Gummi arabicum, Ghatti-Gummi, Karaya-Gummi, Tragant und Agar-Agar. Auch Carboxymethylcellulose, das durch Carboxymethylierung von Zellulose erhalten wird, ist hierfür geeignet.

Als synthetische anionische Polymere können Polymerisate aus einem sauren Vinylmonomeren oder einem seiner Salze eingesetzt werden. Das saure Vinylmonomere ist eine Verbindung, die eine saure Gruppe, z.B. eine Carboxyl-, Sulfonat- oder Phosphat-Gruppe und eine Vinylgruppe pro Molekül enthält. Beispiele hierfür sind ungesättigte Säuren wir die Acryl-, Methacryl-, Croton-, Vinylbenzoe-, 2-Acrylamido-2-methylpropansulfon-, Styrolsulfon-, Vinylsulfon-, Allylsulfon-, Methacrylsulfon- und 3-Methacryl-propansulfonsäure sowie ungesättigte, zweibasische Säuren wie die Itacon-, Malein- und Fumarsäure und deren Monoester. Beispiele für geeignete Salze sind die Natrium-, Kalium- und Ammoniumsalze dieser Säuren.

Zur Copolymerisation kann ein beliebiges anderes Vinylmonomeres, das mit dem sauren Vinylmonomeren copolymerisiert, verwendet werden. Die Menge des anderen Vinylpolymeren sollte jedoch höchstens 60 Mol-%, bezogen auf die gesamte Monomerenmenge, betragen. Dieses andere Vinylmonomere ist eine Vinylverbindung, die mit Hilfe eines radikalischen Initiators polymerisiert. Beispiele hierfür sind Acrylester wie Methylacrylat und Ethylacrylat, Methacrylester wie Methylmethacrylat und Ethylmethacrylat, Styrolverbindungen wie Styrol und Methylstyrol, Acrylamid, Methacrylamid, Vinylether und Vinylacetat.

Als kationische Polymere können kationische Zellulose-Derivate, kationische Stärke, kationische Guar-Gummi-Derivate, Diallyl-quartar-Ammoniumsalz/Acrylamid-copolymere, quartäre Diallylammoniumsalz-polymere, quaternäre Polyvinylpyrrolidon-Derivate und kationische Siliconpolymere verwendet werden.

Als kationische Zellulose-Derivate werden Verbindungen der allgemeinen Formel (III) bevorzugt

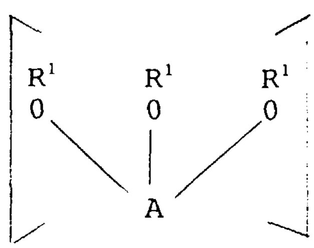

(III)

In Formel (III) steht A für den Rest einer Anhydroglukose-Einheit; f ist eine Zahl zwischen 50 und 20.000 und $R^1$ steht für einen Rest der allgemeinen Formel (III-A)

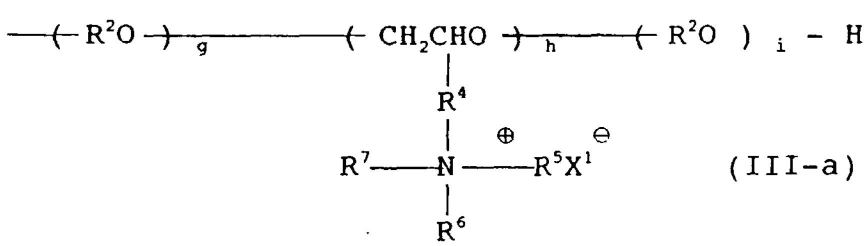

(III-a)

In der allgemeinen Formel (III-a) bedeuten $R^2$ und $R^3$ jeweils eine Alkylengruppe mit 2 oder 3 Kohlenstoffatomen; g ist eine Zahl zwischen 0 und 10; h ist eine Zahl zwischen 0 und 3; und i ist eine Zahl zwischen 0 und 10; $R^4$ bedeutet eine Alkylen- oder Hydroxyalkylengruppe mit 1 bis 3 Kohlenstoffatomen; $R^5$, $R^6$ und $R^7$ sind gleich oder verschieden voneinander und bedeuten eine Alkyl-, Aryl- oder Aralkylgruppe mit bis zu 20 Kohlenstoffatomen oder können auch einen Heterozyklus bilden, der das in der Formel gezeigte Stickstoffatom einschließt; $X^1$ ist ein Anion wie Chlorid, Bromid, Jodid, Sulfat, Sulfonat, Methylsulfonat, Phosphat oder Nitrat.

Der Grad der Substitution des genannten kationischen Zellulose-Derivats reicht von 0,01 bis 1. $R^5$, $R^6$ und $R^7$ können Methylgruppen sein oder eine von diesen Gruppen ist eine langkettige Alkylgruppe mit 10 bis 18 Kohlenstoffatomen, während die übrigen kurzkettige Alkylgruppen mit 1 bis 3 Kohlenstoffatomen sind. Das Molekulargewicht der kationischen Zellulose-Derivate liegt vorzugsweise zwischen 100.000 und 8.000.000.

Die erfindungsgemäß einsetzbare kationische Stärke wird durch die folgende allgemeine Formel (IV) dargestellt

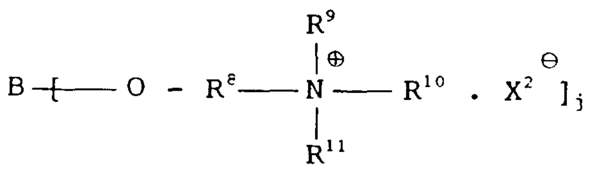

(IV)

In dieser Formel (IV) ist B ein Stärkerest, $R^8$ stellt eine Alkylen- oder Hydroxyalkylengruppe dar, $R^9$, $R^{10}$ und $R^{11}$ sind entweder gleich oder unterscheiden sich voneinander und stellen jeweils eine Alkyl-, Aryl- oder Aralkylgruppe mit bis zu 10 Kohlenstoffatomen dar oder sie bilden einen Heterozyklus unter Einschluß des in der Formel dargestellten Stickstoffatoms; $X^2$ ist ein Anion wie Chlorid, Bromid, Jodid, Sulfat, Sulfonat, Methylsulfat, Phosphat oder Nitrat und $_j$ ist eine positive Zahl.

Die erfindungsgemäß einsetzbaren kationischen Guar-Gummi-Derivate werden durch die allgemeine Formel (V) dargestellt

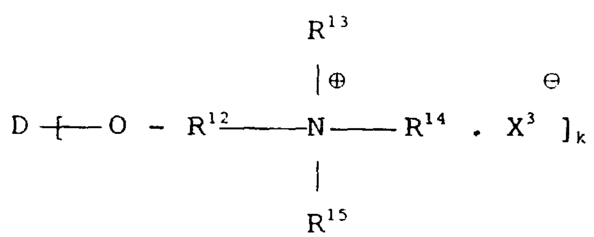

(V)

In dieser Formel (V) bedeutet D den Rest des Guar-Gummis, $R^{12}$ bedeutet einen Alkylen- oder Hydroxyalkylenrest, $R^{13}$, $R^{14}$ und $R^{15}$, können entweder gleich oder verschieden voneinander sein und eine Alkyl-, Aryl- oder Aralkylgruppe mit bis zu 10 Kohlenstoffatomen bedeuten oder sie können einen Heterozyklus zusammen mit dem Stickstoff bilden, der in der Formel dargestellt ist. $X^3$ ist ein Anion wie Chlorid, Bromid, Jodid, Sulfat, Sulfonat, Methylsulfat, Phosphat oder Nitrat; und $_k$ ist eine positive Zahl.

Die erfindungsgemäß vorgesehenen kationischen quartären Diallylammoniumsalzpolymeren und die quartären Diallylammoniumsalz/Acrylamid-copolymeren werden durch die allgemeinen Formeln (VI) und (VII) dargestellt

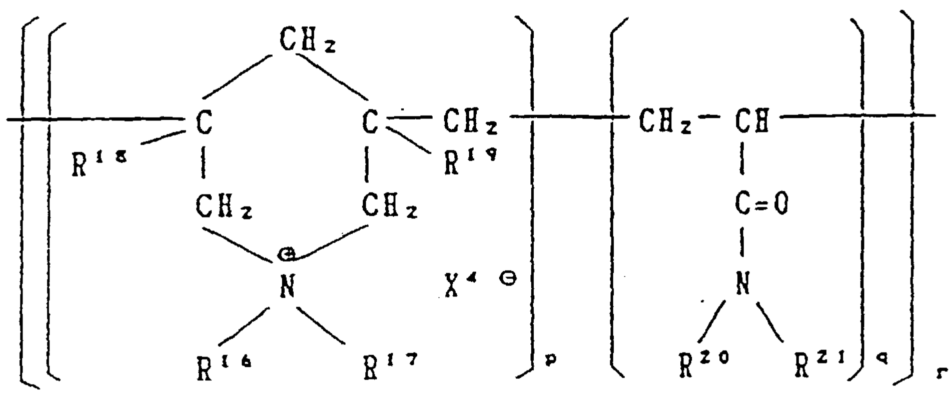

(VI)

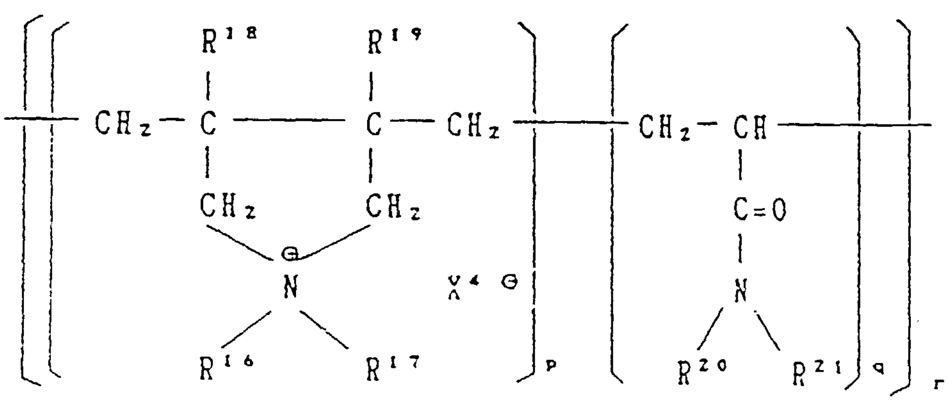

(VII)

In den Formeln (VI) und (VII) sind $R^{16}$ und $R^{17}$ entweder gleich oder unterscheiden sich voneinander und bedeuten jeweils eine Alkylgruppe mit 1 bis 18 Kohlenstoffatomen, Phenyl, Aryl, Hydroxyalkyl, Amidoalkyl, Cyanoalkyl, Alkoxyalkyl oder Carbalkoxyalkylgruppen; $R^{18}$, $R^{19}$, $R^{20}$ und $R^{21}$ können entweder gleich sein oder sich voneinander unterscheiden und Wasserstoffatom oder eine Alkylgruppe mit 1 bis 3 Kohlenstoffatomen oder auch eine Phenylgruppe bedeuten. $X^4$ ist ein Anion wie Chlorid, Bromid, Jodid, Sulfat, Sulfonat, Methylsulfat, Phosphat oder Nitrat, $_p$ ist eine Zahl von 1 bis 50; $_q$ ist eine Zahl zwischen 0 und 50; und $_r$ ist eine Zahl zwischen 150 und 8.000. Das Molekulargewicht der vorstehend genannten Diallylquartär-Ammoniumsalz/Acrylamid-copolymere liegt zwischen etwa 30.000 und 2.000.000, vorzugsweise zwischen 100.000 und 1.000.000.

Die erfindungsgemäß einsetzbaren quaternären Polyvinylpyrrolidon-Derivate werden durch die allgemeine Formel (VIII) dargestellt

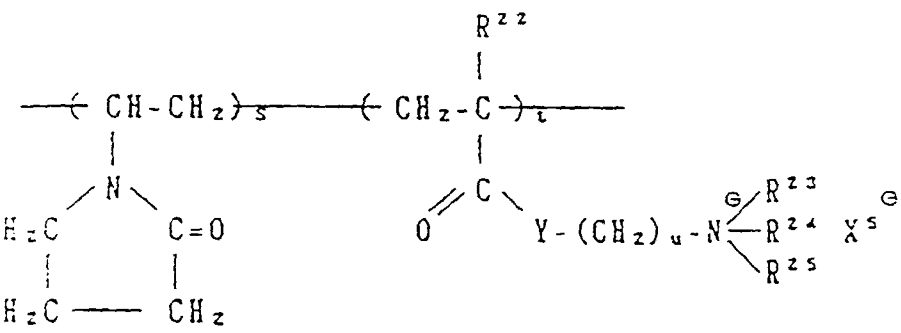

(VIII)

In der Formel (VIII) bedeutet $R^{22}$ ein Wasserstoffatom oder eine Alkylgruppe mit 1 bis 3 Kohlenstoffatomen; $R^{23}$, $R^{24}$ und $R^{25}$ können entweder gleich oder unterschiedlich sein und jeweils ein Wasserstoffatom, eine Alkylgruppe mit 1 bis 4 Kohlenstoffatomen, Hydroxyalkyl, Amidoalkyl, Cyanoalkyl, Alkoxyalkyl oder Carbalkoxyalkylgruppen bedeuten; $_y$ steht für ein Sauerstoffatom oder eine NH-Gruppe in einer Amidbindung; $X^5$ ist ein Anion wie Chlorid, Bromid, Jodid, Sulfat, Sulfonat, Alkylsulfonat mit 1 bis 4 Kohlenstoffatomen, Phosphat oder Nitrat; $_u$ ist eine Zahl zwischen 1 und 10; $_s$ und $_t$ sind Zahlen zwischen 20 und 8.000. Das Molekulargewicht dieser quaternären Polyvinylpyrrolidon-Derivate liegt zwischen 10.000 und 2.000.000, vorzugsweise zwischen 50.000 und 1.500.000.

Typische Beispiele für die erfindungsgemäß einzusetzenden kationischen Siliconpolymere werden durch die allgemeine Formel (IX) dargestellt und haben ein durchschnittliches Molekulargewicht von annäherend 3.000 bis 100.000. Sie tragen die INCI-Bezeichnungen Amodimethicone und Trimethylsilylamodimethicone.

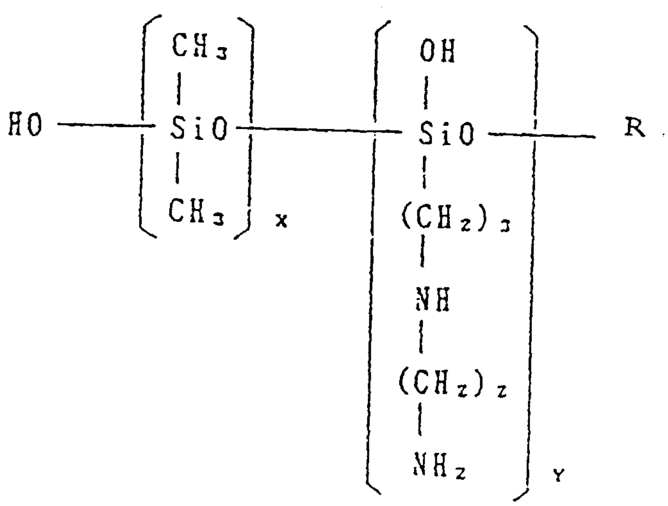

(IX)

wobei R = H oder $(CH_3)_3Si$ bedeuten und die Werte von X und Y vom Molekulargewicht der Verbindung abhängen.

Die vorstehend genannten kationischen Siliconpolymere werden vorzugsweise in Form einer wäßrigen Emulsion eingesetzt. Ihre Herstellung ist in dem U.S.Patent No.4,908,140 beschrieben.

Das erfindungsgemäß einzusetzende amphotere Polymere kann durch Polymerisation eines sauren Vinylmonomeren mit einem basischen Vinylmonomeren, durch Polymerisation eines amphotehergestellt werden. Typische Beispiele für diese amphoteren Polymeren sind folgende:

1. **Copolymeres aus einem sauren und aus einem basischen Vinylmonomeren:**

Ein typisches Beispiel hierfür ist ein amphoteres Copolymeres, das durch die Polymerisation einer Monomeren-Mischung, die 45 bis 55 Mol-% eines sauren Vinylmonomeren oder seines Salzes und 45 bis 55 Mol-% eines basischen Vinylmonomeren oder seines Salzes enthält, in Gegenwart eines bekannten Initiatiors für Radikalpolymerisationen und bei einer Temperatur von etwa 150°C hergestellt wird. Dieses molare Verhältnis gilt dann, wenn die beiden miteinander reagierenden Vinylmonomeren jeweils eine saure und eine basische Gruppe pro Molekül erhalten. Beispiele für saure Vinylmonomere sind Acrylsäure, Methacrylsäure, Crotonsäure, Vinylbenzoesäure, Vinylsulfonsäure aber auch zweibasische Säuren wie Malein- und Fumarsäure und ihre Monoester.

Basische Vinylmonomere sind Verbindungen, die eine primäre, sekundäre oder tertiäre Aminogruppe und eine polymerisierbare Vinylgruppe pro Molekül enthalten. Beispiele hierfür sind Dimethylaminoethyl-methacrylat, Dimethylaminoethyl-methacrylat, Dimethylaminoethyl-acrylat, Diethylaminoethyl-acrylat, Dimethylaminopropyl-acrylat, Dimethylaminopropyl-methacrylamid, 2-Vinylpyridin, 4-Vinylpyridin, Dimethylallylamin und ihre Salze.

2. **Polymere aus einem amphoteren Monomeren**

Typische Beispiele hierfür sind amphotere Polymere, die durch Polymerisation eines amphoteren Monomeren der Formel (X) in Gegenwart eines Initiatiors für eine Radikalpolymerisation bei einer Temperatur zwischen 20 und 130°C hergestellt werden können.

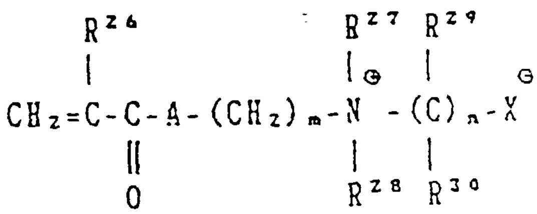

(X)

in der $R^{26}$, $R^{29}$ und $R^{30}$ jeweils Wasserstoff oder eine Methylgruppe sind; $R^{27}$ und $R^{28}$ eine Methyl oder Ethylgruppe bedeuten; A = -O- oder -NH ist; X = $-CO_2$, $-SO_3$ oder $-PHO_3$ bedeuten und $_m$ und $_n$ Zahlen zwischen 1 und 3 sind.

Die amphoteren Monomeren gemäß Formel (X) werden durch eine Reaktion eines geeigneten Aminoalkylacrylats oder eines Aminoalkylamids mit einem Lacton, Sulton oder einem zyklischen Phosphid hergestellt.

Jedes der vorstehend genannten anionischen, kationischen oder amphoteren Polymere kann allein oder in Kombination mit anderen Polymeren verwendet werden. Die Polymeren werden im allgemeinen in Mengen von 0,01 bis 10 Gewichts-%, vorzugsweise in Mengen von 0,1 bis 5 Gewichts-%, bezogen auf die Gesamtmenge des erfindungsgemäßen Haarpflegemittels, eingesetzt. Ist ihre Menge kleiner als 0,01 Gewichts-%, dann können keine konditionierenden Effekte mehr beobachtet werden. Liegt die Menge des Polymeren über 10 Gewichts-%, dann kann andererseits keine Verbesserung der konditionierenden Wirkungen mehr festgestellt werden.

Das erfindungsgemäße Haarpflegemittel kann außer dem Carrier und dem kosmetisch akzeptablen, kationischen, anionischen oder amphoteren Polymeren auch noch weitere, für Haarpflegeprodukte übliche Inhaltsstoffe enthalten, zum Beispiel Konsistenzgeber (Fettalkohole), Öle und Wachse (Triglyzeride, Paraffinöl, Siliconöl-Derivate, Wollwachs, Bienenwachs, Fruchtwachse), Haarpflegestoffe (Panthenol, Vitamine, Zucker, Biotin, Proteine), Verdicker, organische Säuren, Konservierungsmittel und Parfüme.

Das erfindungsgemäße Haarpflegemittel liegt als eine Öl/Wasseremulsion vor, die aber auch als Aerosol versprüht werden kann und ist auf einen pH 2 bis 8 eingestellt.

Durch die folgenden Beispiele und Vergleichsbeispiele wird das erfindungsgemäße Haarpflegemittel noch weiter verdeutlicht.

Tabelle 1

| Ansatz Nr. | V1 | V2 | V3 | V4 | E1 | E2 |
|---|---|---|---|---|---|---|
| Cetearylalkohol 50:50 (Lanette O) | 4,0 | 4,0 | 4,0 | 4,0 | 4,0 | 4,0 |
| Cetrimonium-chlorid | 0,5 | 0,5 | 0,5 | 0,5 | 0,5 | 0,5 |
| Glykolsäure | 0,5 | 0,5 | 0,5 | 0,5 | 0,5 | 0,5 |
| Vanillin | - | 3,0 | - | - | 3,0 | 3,0 |
| Phenoxyethanol | - | 1,0 | - | - | 1,0 | 1,0 |
| Amodimethicone* | - | - | - | 2,0 | - | 2,0 |
| Trimethylsilyl-amodimethicone** | - | - | 2,0 | - | 2,0 | - |
| Parfüm | 0,5 | 0,5 | 0,5 | 0,5 | 0,5 | 0,5 |
| Wasser | ad 100 | ad 100 | ad 100 | ad 100 | ad 100 | ad 100 |
| pH | 3,0 | 3,0 | 3,0 | 2,9 | 3,0 | 3,0 |

*enthalten in dem Handelspräparat Dow Corning 939
**enthalten in dem Handelspräparat Dow Corning Q2-7224

V1 und V2 sind sind Vergleichsrezepturen, die kein kationisches oder amphoteres Polymeres enthalten. V3 und V4 sind Vergleichsrezepturen, die keinen Carrier enthalten, wobei V3 mit E1 und V4 mit E2 verglichen werden (gleiche Basis, gleiches Polymer). E1 und E 2 sind die erfindungsgemäßen Rezepturen.

Die Herstellung des erfindungsgemäßen Haarpflegemittels erfolgt nach einem an sich bekannten verfahren: die 80°C warme, wäßrige Lösung der wasserlöslichen Rohstoffe wird in die bei 80°C geschmolzene Wachsphase eingerüht, die Emulsion dann bei 80°C 5 Minuten emulgiert, ggf. homogenisiert und unter gelegentlichem Rühren abgekühlt; bei ca. 30°C werden das Parfüm und die Polymer-Emulsion eingearbeitet und die Wasser-Verdampfungsverluste ausgeglichen.

Die Wirkung des erfindungsgemäßen Haarpflegemittels auf Haare wurde durch Kämmkraftmessungen mit einer Kämmaschine nachgewiesen. Hierzu wurden gebleichte Haarsträhnen mit einem 0-Shampoo gewaschen. Der O-Shampoo hat folgende Zusammensetzung:

| | |
|---|---|
| 40g | Texapon N25 SAL (INCI-Name: Natrium-Laurethsulfat(28%), Salizylsäure(0,3%) in Wasser), |
| 4g | Natriumchlorid |
| ad 100g | Wasser |

Danach wurden die Haare mit dem zu untersuchenden Haarpflegemittel 20 Minuten bei 40°C behandelt. Anschließend wurde das Haarpflegemittel ausgespült und die feuchten Haarsträhnen gekämmt. Dann wurde jede Haarsträhne wieder mit 0-Shampoo gewaschen und feucht gekämmt. Dieser Vorgang wurde solange wiederholt, bis die Kämmharkeit jeweils wieder den Wert der unbehandelten Strähnen erreicht hatte. Die Kämmkräfte (in [N]) nach der jeweiligen Behandlung des untersuchten Haarpflegemittels sind in der folgenden Tabelle dargestellt; fett gedruckte Werte unterscheiden sich signifikant von denen der unbehandelten Strähnen:

Tabelle 2

| Behandlung | unbehandelt | V1 | V2 | V3 | V4 | E1 | E2 |
|---|---|---|---|---|---|---|---|
| Anwendung der Kur | 0.638 | 0.329 | 0.320 | 0.151 | 0.186 | 0.177 | 0.186 |
| 1. Wäsche | 0.976 | 1.014 | 0.909 | 0.283 | 0.565 | 0.365 | 0.482 |
| 2. Wäsche | 1.185 | | | 0.417 | 0.663 | 0.371 | 0.485 |
| 3. Wäsche | 1.017 | | | 0.438 | 0.711 | 0.461 | 0.536 |
| 4. Wäsche | 1.022 | | | 0.517 | 0.959 | 0.656 | 0.723 |
| 5. Wäsche | 1.220 | | | 0.640 | | 0.635 | 0.955 |

Tabelle 2 (fortgesetzt)

| Behandlung | unbehandelt | V1 | V2 | V3 | V4 | E1 | E2 |
|---|---|---|---|---|---|---|---|
| 6. Wäsche | 1.256 | | | 0.847 | | 0.762 | |
| 7. Wäsche | 1.165 | | | 1.073 | | 0.809 | |
| 8. Wäsche | 1.159 | | | | | 0.980 | |

**Patentansprüche**

**1.** Haarpflegemittel mit langdauernder Wirkung enthaltend in einer kosmetisch anwendbaren Basiszubereitung

a) 0,1 bis 20 Gewichts-% eines Carriers, der mindestens eine Hydroxylgruppe im Molekül aufweist, bei einem pH-Wert von 7,0 ungeladen ist, einen Octanol-Wasser-Verteilungskoeffizienten (log P) von 0,3 bis 3,0 besitzt, jedoch nicht 2-Benzyloxyethanol ist und

b) 0,1 bis 20 Gewichts-% eines oder mehrerer kosmetisch akzeptabler, anionischer, kationischer oder amphoterer Polymerer.

**2.** Mittel nach Anspruch 1, **dadurch gekennzeichnet**, daß der Carrier aus Verbindungen der Formel (I)

$$(R)_p - Z - X - Y - OH \qquad (I)$$

ausgewählt ist, wobei X eine $(CH_2)_n$-Gruppe mit n 0; 1 oder 2, eine Alkoxygruppe, eine Hydroxyalkylgruppe, Sauerstoff oder Schwefel bedeutet, Y eine $(CH_2)_m$-Gruppe mit m = 0, 1 oder 2 bedeutet; Z einen 5- bis 8-gliedrigen aliphatischen oder aromatischen Carbozyklus oder Heterozyklus bedeutet und (R)p für bis zu fünf Substituenten (p = 0, 1, 2, 3, 4 oder 5) steht, Welche unabhängig voneinander Wasserstoff, eine Hydroxygruppe, eine Alkylgruppe, , eine Alkoxygruppe, eine Halogenalkylgruppe, Halogen, eine Acetylgruppe, eine Acetamidogruppe, eine Formylgruppe oder eine Formylalkylgruppe darstellen, unter der Voraussetzung, daß die Verbindung der Formel (I) nicht gleich 2-Benzyloxyethanol ist.

**3.** Mittel nach Anspruch 1, **dadurch gekennzeichnet**, daß der Carrier ausgewählt ist aus α-Hydroxycarbonsäureeestern der Formel (II)

$$R^1 - CH(OH) - COOR^2 \qquad (II),$$

mit $R^1$ und $R^2$ unabhängig voneinander gleich einer Alkylgruppe mit 1 bis 6 Hohlenstoffatomen, wobei die Alkylgruppe durch 1 oder 2 Sauerstoffatome mit Etherfunktion unterbrochen sein kann.

**4.** Mittel nach einem der Ansprüche 1 bis 3, **dadurch gekennzeichnet**, daß der Carrier ausgewählt ist aus Vanillin, p-Hydroxyanisol, 3-Hydroxy-4-methoxybenzaldehyd, 2-Phenoxyethanol, Salicylaldehyd, 3,5-Dihydroxybenzaldehyd, 3,4-Dihydroxybenzaldehyd, 4-Hydroxy-phenylacetamid, p-Hydroxybenzoesäuremethylester, p-Hydroxybenzaldehyd, m-Kresol, Hydrochinonmonomethylether, o-Fluorphenol, m-Fluorphenol, p-Fluorphenol, 2-(2'-Hydroxyphenoxy)ethanol, 3,4-Methylendioxyphenol, Resorcin-monomethylether, 3,4-Dimethoxy-phenol, 3-Trifluormethyl-phenol, Resorcin-monoacetat, Ethylvanillin, 2-Thiophenethanol, Milchsäurebutylester und Glykolsäurebutylester oder Mischungen der vorgenannten Verbindungen.

**5.** Mittel nach einem der Ansprüche 1 bis 4, **dadurch gekennzeichnet**, daß der Carrier einen Octanol-Wasser-Verteilungskoeffizienten (log P) von 0,5 bis 2,7.aufweist.

**6.** Mittel nach Anspruch 1, **dadurch gekennzeichnet**, daß das kosmetisch akzeptable Polymere aus der Gruppe der amino-funktionellen Siliconpolymeren ausgewählt ist.

**7.** Mittel nach Anspruch 1, **dadurch gekennzeichnet**, daß es einen pH-Wert von 2 - 8 aufweist.

**8.** Verfahren zur Pflege von Haaren, **dadurch gekennzeichnet**, daß das Haarpflegemittel nach einem der Ansprüche 1 - 7 in einer Menge von 5 - 50 g auf die Haare aufgetragen und das Haar nach einer Einwirkungszeit von 2 - 60 min bei 15 - 50'C mit Wasser ausgespült und getrocknet wird.